(19) Europäisches Patentamt
European Patent Office
Office européen des brevets

(11) **EP 3 233 102 B1**

(12) **EUROPEAN PATENT SPECIFICATION**

(45) Date of publication and mention
of the grant of the patent:
**06.03.2024 Bulletin 2024/10**

(21) Application number: **14841349.5**

(22) Date of filing: **23.12.2014**

(51) International Patent Classification (IPC):
**A61K 38/00** (2006.01)     **C07K 7/00** (2006.01)

(52) Cooperative Patent Classification (CPC):
(C-Sets available)
**C07K 7/08; A61K 31/407; A61K 31/5365;
A61K 31/555; A61K 31/704; A61K 31/7048;
A61K 33/243; A61K 38/10; A61K 45/06**     (Cont.)

(86) International application number:
**PCT/TR2014/000509**

(87) International publication number:
**WO 2016/099409 (23.06.2016 Gazette 2016/25)**

(54) **USE OF AN ANTIMICROBIAL PEPTIDE TO INDUCE CELL DEATH IN BREAST CANCER**

VERWENDUNG EINES ANTIMIKROBIELLEN PEPTIDS ZUR INDUKTION DES ZELLTODES BEI
BRUSTKREBS

UTILISATION D'UN PEPTIDE ANTIMICROBIENS POUR INDUIRE LA MORT CELLULAIRE DANS
LE CANCER DU SEIN

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **19.12.2014 TR 201415474**

(43) Date of publication of application:
**25.10.2017 Bulletin 2017/43**

(73) Proprietors:
• **Bolkent, Sehnaz**
  **34134 Istanbul (TR)**
• **Sancar Bas, Serap**
  **34134 Istanbul (TR)**
• **Istanbul University**
  **34452 Beyazit, Istanbul (TR)**

(72) Inventors:
• **BOLKENT, Sehnaz**
  **Biyoloji Bölümü Zooloji Anabilim Dali (TR)**
• **SANCAR BAS, Serap**
  **Biyoloji Bölümü Zooloji Anabilim Dali (TR)**

(74) Representative: **Kaya, Erdem**
**Erdem Kaya Patent Inc.**
**Konak Mahallesi Kudret Sk.**
**Elitpark Park Sit. Ofisler Apt.**
**No: 12/27**
**16110 Nilüfer/Bursa (TR)**

(56) References cited:
**WO-A2-99/60016**

• **DIANA GASPAR ET AL: "From antimicrobial to
anticancer peptides. A review", FRONTIERS IN
MICROBIOLOGY, vol. 4(294), 1 October 2013
(2013-10-01), pages 1-16, XP055210470, DOI:
10.3389/fmicb.2013.00294**
• **HOSKIN ET AL: "Studies on anticancer activities
of antimicrobial peptides", BIOCHIMICA ET
BIOPHYSICA ACTA (BBA) - BIOMEMBRANES,
ELSEVIER, AMSTERDAM, NL, vol. 1778, no. 2, 22
November 2007 (2007-11-22), pages 357-375,
XP022428150, ISSN: 0005-2736, DOI:
10.1016/J.BBAMEM.2007.11.008**

**EP 3 233 102 B1**

- **JAMIE S MADER ET AL: "Cationic antimicrobial peptides as novel cytotoxic agents for cancer treatment", EXPERT OPINION ON INVESTIGATIONAL DRUGS, vol. 15, no. 8, 1 August 2006 (2006-08-01), pages 933-946, XP055059217, ISSN: 1354-3784, DOI: 10.1517/13543784.15.8.933**
- **RADERMACHER S W ET AL: "Bactenecin, a leukocytic antimicrobial peptide, is cytotoxic to neuronal and glial cells", JOURNAL OF NEUROSCIENCE RESEARCH, WILEY-LISS, US, vol. 36, no. 6, 15 December 1993 (1993-12-15), pages 657-662, XP002125625, ISSN: 0360-4012, DOI: 10.1002/JNR.490360606**

Remarks:

The file contains technical information submitted after the application was filed and not included in this specification

(52) Cooperative Patent Classification (CPC): (Cont.)

C-Sets
**A61K 31/407, A61K 2300/00;**
**A61K 31/5365, A61K 2300/00;**
**A61K 31/555, A61K 2300/00;**
**A61K 31/704, A61K 2300/00;**
**A61K 31/7048, A61K 2300/00;**
**A61K 33/24, A61K 2300/00;**
**A61K 38/10, A61K 2300/00**

Remarks:

**Description**

FIELD OF THE INVENTION

The present invention relates to use of a cyclic dodecapeptide having the sequence of sequence ID No: 1 wherein said peptides comprises a disulfide bond formed between the two cysteine residues to induce cell death in breast cancer.

BACKGROUND

Antimicrobial peptides are molecules which play important roles in the natural immune system from insects to mammals. It has been stated that these peptides modulate the immune system in inflammation and infections, neutralize lipopolysaccharides (LPS), and are effective against Gram positive and Gram negative bacteria by regulating adaptive immune responses. Antimicrobial peptides show significant differences in terms of amino acid sequence and secondary structure. The net charges of the most of these peptides vary between +2 to +9 in neutral pH. This cationic character of the antimicrobial peptides is originated from basic amino acids that they have such as lysine and arginine. On the other hand, these peptides are short peptides comprising 5-40 amino acids and 30% or more of amino acids have hydrophobic characters.

The antibiotics used today target intracellular specific proteins. The cationic antimicrobial peptides generate pores or deteriorate lipid layers, and thus destroy cell membranes and cause cell lysis and death. The negative charge of the bacteria membrane is the most important factor creating this effect. The bacteria membranes arc rich in negatively charged lipids such as phosphatidylglycerol, cardiolipin or phosphatidylserine, and the electrostatic interaction of positive charged cationic antimicrobial peptides with these lipids causes the cationic peptides to bond to the bacteria membrane. Furthermore, the outer membrane in the Gram negative bacteria is negative charged since it comprises anionic lipopolysaccharide. It has been stated that some antimicrobial peptides pass through the outer membrane of the Gram negative bacteria, settle in the cytoplasmic membrane, and they have the characteristics to thin the membrane, form pores, and deteriorate the barrier function of the membrane or affect intracellular targets by passing the membrane. The amphipathic characteristic which is an important criterion in displaying antimicrobial effect is induced by the secondary structure which the peptides have at the time when they interact with the membrane. The antimicrobial peptides exhibit different secondary structures such as a-helix, β-sheet, loop or linear.

Aside from amphipathicity, physicochemical characteristics such as the amino acid sequence, hydrophobicity, net charge, the structural folding during interaction with membrane, membrane composition, oligomerization, and peptide concentration play a role in occurrence of antimicrobial effect. On the other hand, mammalian cell membrane comprises plenty of zwitterionic phospholipids such as phosphatidyl ethanol amine, phosphatidylcholine, and sphyngomyelin, for this reason, normal cellular membranes are less attractive for cationic antimicrobial peptides. Furthermore, the cholesterol in the mammalian cell membranes makes difficult to deteriorate the lipid bilayer by the antimicrobial peptides. These characteristics make the antimicrobial peptides more selective against bacteria.

Beside bacteria killing properties, it has been stated that the peptides have some natural immune functions. These functions are chemotaxis of neutrophils, mast cells and monocytes, stimulating phagocytosis, wound healing and angiogenesis, stimulating the generation of chemokines such as IL-8 and MCP- 1 from epithelium cells and macrophages, and releasing secretions of the mast cells. These activities may vary between different antimicrobial peptides. For this reason, the peptides have the characteristics to manage natural immune responses, eliminate pathogens and improve wound healing.

A cyclic dodecapeptide defined firstly in the neutrophils of bovine (Bos taurus) and is 12-mer peptide which is Arg-Leu-Cys-Arg-Ile-Val-Val-Ilc-Arg-Val-Cys-Arg. Its secondary structure is a loop. It has 66% hydrophobicity, and +4 net charge. The antibacterial effect of this material was researched with colony forming method and it was observed that it is more effective on Gram negative bacteria. Furthermore, it was observed that it permeabilizes the inner membrane in E. coli bacteria without outer membrane by using fluorescent dye.

In a publication by Gaspar et. al. "From antimicrobial to anticancer peptide. A review", Fromtiers in Microbiology, 4(294), 2013, 1-16; it was disclosed that several peptides are effective against solid and hematological tumors both in vitro and in vivo. It was also disclosed that some peptides Show both antimicrobial and anticancer properties however with different modes of action.

In another publication by Hoskin et. al. "Studies on anticancer activities of antimicrobial peptides", Biochimica et Biophysica Acta (BBA) - Biomembranes, 1778(2), 2007. 357-375; it was disclosed that positively charged antimicrobial peptides were able to show cytotoxic activity against cancer cells probably due to the strong binding between the negatively charged components of the cancer cell and the positively charged antimicrobial peptides.

A similar publication by Mader et. al. "Cationic antimicrobial peptides as novel cytotoxic agents for cancer treatment", Expert Opnion on Investigational Drugs. 15(8), 2006, 933-946: investigates the use of cationic antimicrobial peptides as an alternative to traditional chemotherapy treatment and mechanisms of action involved in their activity. It was further disclosed that some of the cationic antiocrobial peptides have angiogenesis inhibiting properties.

A patent application numbered WO 99/60016 was made in the same general field of cationic antimicrobial peptides. In particular said document discloses antimicrobial peptide bactenecin and derivatives thereof and investigates the effect said peptides on intibiting cell-proliferation associated disorders such as cancer.

Another publication by Radermacher et. al. "Bactenecin, a leukocytic antimicrobial peptide, is cytotoxic to neuronal and glial cells". Journal of Neuroscience Research, 36(6), 1993, 657-662 discloses that bactenecin, which is a docecapeptide, is strongly cytotoxic to emryonic neurons of rats and human glioblastoma cells. It was also found that of all the positively charged peptides only bactenecin show said cytotoxic effect.

SUMMARY OF THE INVENTION

The present invention is defined by the appended claims. Inventions, embodiments and aspects mentioned in the description, but no longer falling under the scope of said claims are considered merely for understanding the present invention.

The present invention relates to the medical use in the treatment of breast cancer of a cyclic dodecapeptide having the sequence ID No: 1 and comprising a disulfide between the two cysteine residues as defined in claim 1, i.e. the invention features the medical use of inducing cell death of cancerous cells of breast cancer. Accordingly, the invention includes the in vivo administration of a therapeutically effective amount of a peptide of the sequence SEQ ID NO: 1 to cancerous cells.

In other embodiments, the peptide forms a parallel or an anti-parallel dimer with a second peptide and the dimer is a homo- or a hetero-dimer.

In some embodiments, one or more cysteine residues in the peptide are substituted with serine residues.

In other embodiments, one or more serine residues in the peptide are substituted with cysteine residues.

The peptide induces cell death of cancerous cells (while in certain embodiments not inducing cell death in corresponding normal cells), e.g., by inducing necrotic cell death through deteriorating cell membrane integrity.

In other embodiments, the peptide is synthesized using an automatic peptide synthesizer and purified using high-performace liquid chromatography (HPLC) to a purity of at least 95 %.

In some embodiments, the method of the first or second aspect of the invention decreases cell viablility of the cancerous cells by at least 50%.

In some embodiments, the therapeutically effective amount of the peptide in the first or second aspect of the invention is from 0.001 to 500 mg kg/day.

In vivo means in a mammal. preferably the mammal is a human.

In other embodiments, the method of the first or second aspect of the invention further includes administering a chemotherapeutic agent together with the peptide. In particular, the chemotherapeutic agent can be selected from, e.g., the group consisting of cisplatin, carboplatin, oxaliplatin, bleomycin, mitomycin C, calicleamieins, maytansinoids, doxorubicin, idarubicin, daunorubicin, epirubicin, busulfan, carmustine, lomustine, semustine, thalidomide, lenalidomide, methotrexate, 6-mercaptopurine, fludarabinc, 5-azacytidine, pentostatin. cytarabine, gemcitabine, 5-fluorouracil, hydroxyurea, etoposide, teniposide, topotecan, irinotecan, chlorambucil, cyclophosphamide, ifosfamide, melphalan, bortezomib, vincristine, vinblastine, vinorelbine, paclitaxel, and docetaxel.

In some embodiments, the pharmaceutical composition includes one or more pharmaceutically acceptable carriers or excipients. In certain embodiments, the peptide is not naturally occuring. In certain preferred embodiments, the peptide (e.g., a peptide having a sequence of SEQ ID NO: 1) has cyclic dodecapeptide activity.

Definitions

As used herein, the term "basic amino acid" refers to an amino acid that has an overall positive charge at a neutral pH (i.e., a pH of 7). A basic amino acid, when incorporated into a peptide or protein, contributes at least a positive charge of one to the peptide or protein. Within the naturally occurring amino acids, the basic amino acids are histidine, arginine, and lysine.

As used herein, the term 'hydrophobic amino acid" refers to an amino acid that is non-polar and prefers to interact with other neutral molecules and non-polar solvents. Within the naturally occurring amino acids, the hydrophobic amino acids are isoleucine, alanine, phenylalanine, valine, proline, tryptophan, and methionine.

As used herein, the term "cyclic" refers to a form of the peptide used in the methods and pharmaceutical compositions of the invention. To form a cyclic peptide, the peptide may contain intra-chain disulfide bonds in which one cysteine residue within the peptide covalently bonds with another cysteine residue within the same peptide.

As used herein, the term "therapeutically effective amount" or "effective amount" refers to an amount effective to achieve the desired therapeutic effect while avoiding adverse side effects, e.g., the amount of the peptide used in the methods and pharmaceutical compositions of the invention that is effective in inducing cell death of at least 50% (e.g., 50%, 55%, 60%, 65%, 70, 75%, 80%, 85%, 90%, 92%, 95%, 97%, 99%, 100%), preferably at least 75%, of cancerous cells in vitro or in vivo, while the effect is not observed in normal, healthy cells. In some embodiments, the effective amount of the peptide may be able to eliminate the tumor or reduce tumor size. The effective amount of the peptide may change depending on the type of cancer being treated, e.g., breast cancer.

A peptide is said to have "cyclic dodecapeptide activity" if, when administered to a population of cancer cells (e.g., MCF-7 and/or MDA-MB 231 cells), the peptide is toxic (e.g., by inducing necrotic cell death) to the cancer cells at a concentration at which the peptide is nor toxic to corresponding normal cells (e.g., fibroblasts).

DESCRIPTION OF THE DRAWINGS

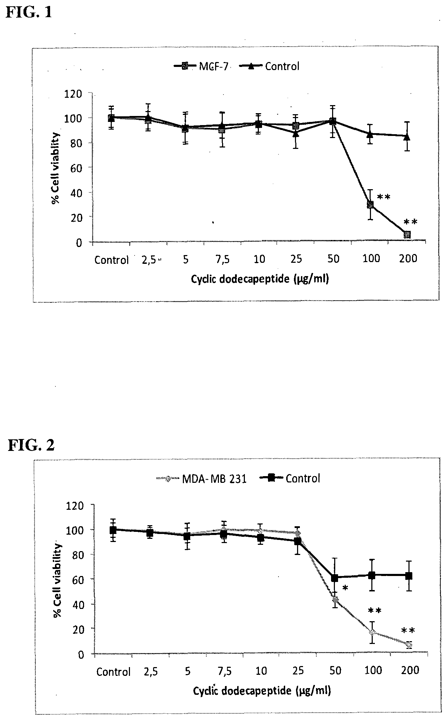

FIG. 1 is a graph showing the effect of cyclic dodecapeptide (SEQ ID NO: 1) on the cell viability of MCF-7 breast cancer cells. MCF-7 breast cancer cells treated with the cyclic dodecapeptide exhibited significant decrease in cell viability when compared to control group of normal cells (** p<0,001).

Percentage of viable MCF-7 cells in the presence of increasing concentrations of cyclic dodecapeptide: Control: 100±7.5, 2.5 μg/ml: 98.2±7.6, 5 μg/ml: 90.84+12, 7.5 μg/ml ;90.31+14.2, 10 μg/ml: 94.2+8.2, 25 μg/ml: 92.98±8.7, 50 μg/ml: 96.37±13, 100 μg/ml: 28.76±12.2, 200 μg/ml: 4.75±1.

Percentage of viable normal cells in the presence of increasing concentrations of cyclic dodecapeptide: Control (not containing DMSO): 100+9.4, 2.5 μg/ml 100.27±11.1, 5 μg/ml: 92.11±11.9 7.5 μg/ml: 93.61±10.1. 10 μg/ml 94.67±6.7, 25 μg/ml: 86.82±12.5, 50 μg/ml: 96.84±9.5, 100 μg/ml: 85.6±8, 200 μg/ml: 83.6±11.7.

FIG. 2 is a graph showing the effect of cyclic dodecapeptide (SEQ ID No: 1) on the cell viability of MDA-MB 231 breast cancer cells. MDA-MB 231 breast cancer cells treated with the cyclic dodecapeptide exhibited significant decrease in cell viability when compared to control group of normal (noncancerous) cells (* p<0,05; **p<0,001). Percentage of viable MDA-MB 231 cells in the presence of increasing concentrations of cyclic dodecapeptide: Control: 100±5.6. 2.5 μg/ml: 98.6±5.1, 5 μg/ml: 95.51±5.8, 7.5 μg/ml: 99.6±6.8, 10 μg/ml: 98.62±5.5, 25 μg/ml: 96.66±5.2, 50 μg/ml: 42.54±6.3, 100 μg/ml: 16.17±8.6, 200 μg/ml: 6.24±2.8. Percentage of viable normal cells in the presence of increasing concentrations of cyclic dodecapeptide: Control (not containingDMSO): 100±8.8,2.5 μg/ml: 97.73±4.6, 5 μg/ml: 94.6±10.3. 7.5 μg/ml: 96.3±7, 10 μg/ml: 93.11±5.3, 25 μg/ml: 90.22±11. 50 μg/ml: 60.2±15.6, 100 μg/ml: 61.99±12.4, 200 μg/ml: 61.71±12.2.

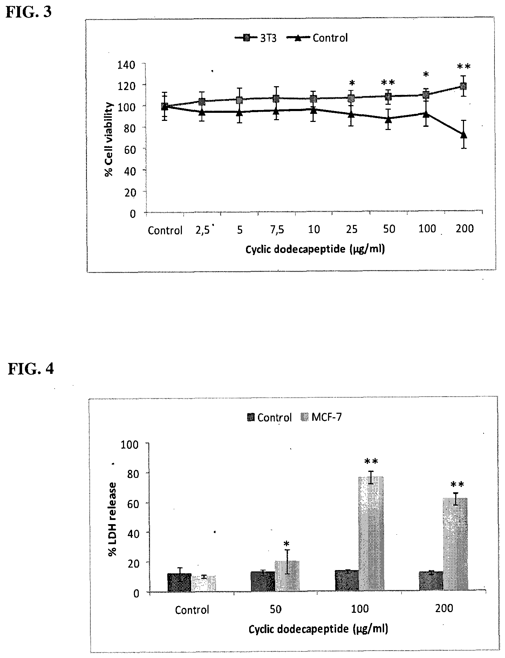

FIG. 3 is a graph showing the effect of cyclic dodecapeptide (SEQ ID NO: 1) on cell viability in 3T3 fibroblast cells. 3T3 fibroblast cells treated with the cyclic dodecapeptide exhibited significant difference in cell viability when compared control group of normal (non-cancerous) cells (* p<0.05; ** p<0.001). Percentage of viable 3T3 fibroblast cells in the presence of increasing concentrations of cyclic dodecapeptide: Control: 100±13.2, 2.5 pg/ml: 104.2±9.7, 5 μg/ml: 105.78±11.1, 7.5 μg/ml: 106.8±10.9, 10 μg/ml 106.3+7.2, 25 μg/ml: 107.2±7.6, 50 μg/ml: 107.9±6.9. 100 μg/ml: 109.33±5.7, 200 μg/ml: 117.55±9.4.

Percentage of viable normal cells in the presence of increasing concentrations of cyclic dodecapeptide: Control (not containing DMSO): 100±9.4, 2.5 pg/ml: 94.74±8.8, 5 μg/ml. 94.21±10.2, 7.5 μg/ml 95.36±8, 10 μg/ml 95.87±10.5, 25 μg/ml 91.97±11.7, 50 μg/ml 86.6±9.4. 100 μg/ml 91.34±11.9, 200 μg/ml 71.78±13.1.

FIG. 4 is a graph showing the effect of cyclic dodecapeptide on (the release of) lactate dehydrogenase in MCF-7 cells. Significant increase in LDH release was observed when compared to control groups (*p<0.05** p<0.001). Values of control group: Control: 12.08±4.4. 50 μg/ml: 12.8±1.9, 100 μg/ml: 13.36±1.5; 200 g/ml: 12.07±1.4; Values of MCF-7 group: Control: 10.29+0.7, 50 μg/ml: 20.11±8.3, 100 μg/ml 76.22±4.1; 200 μg/ml 61.55+4.

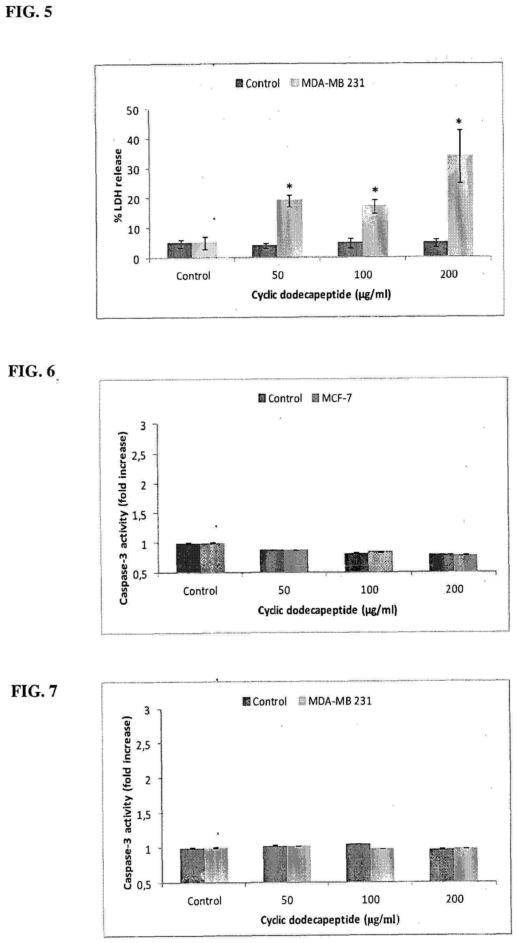

FIG. 5 is a graph showing the effect of cyclic dodecapeptide on lactate dehydrogenase in MDA-MB 231 cells. Significant increase in LDH release was observed when compared to control group (* p<0.05). Values of control group: Control: 4.69±1.2, 50 μg/ml 3.9±0.9, 100 g/ml: 4.73±1.7; 200 μg/ml: 4.8±1.3; Values of MDA-MB 231 group: Control: 4.89±2.2. 50 μg/ml: 19.1±1.9, 100 μg/ml 17.1±2.2; 200 μg/ml 33.93±9.

FIG. 6 is a graph showing the effect of cyclic dodecapeptide on caspase-3 activity in MCF-7 cells. No significant difference was observed in caspase-3 activity when compared to control groups (p>0.05). Values of control group: Control: 1±0.007, 50 μg/ml 0.88±0.005, 100 μg/ml 0.82±0.004, 200 μg/ml: 0.8±0.004. Values of MCF-7 group: Control: 1±0.007, 50 μg/ml: 0.88+0.007, 100 μg/ml: 0.84±0.005, 200 μg/ml: 0.79±0.004.

FIG. 7 is a graph showing the effect of cyclic dodecapeptide on caspase-3 activity in MDA-MB 231 cells. No significant difference was observed in caspase-3 activity when compared to control groups (p>0.05). Values of control group: Control: 1±0.001. 50 μg/ml: 1.028±0.004. 100 μg/ml: 1.05±0.0016, 200 μg/ml: 0.97±0.003. Values ofMDA-MB 231: Control: 1±0.001, 50 μg/ml :1.02±0.005, 100 μg/ml: 0.98 ±0.002, 200μg/ml: 0.98±0.002.

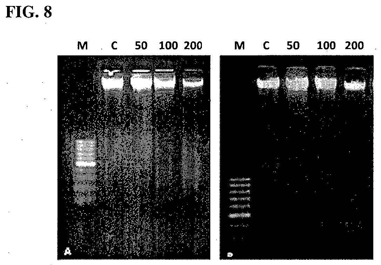

FIG. 8 is an image showing the effect of cyclic dodecapeptide on DNA fragmentation. A) MCF-7 cells and B) MDA-MB 231 cells were incubated with different concentrations of cyclic dodecapeptide such as 50, 100 and 200 μg/ml for 24 hours. DNA agarose gel electrophoresis showed that there is no fragmentation in DNA isolated from the cells (M: 100 bp marker; K: DNA of the control group).

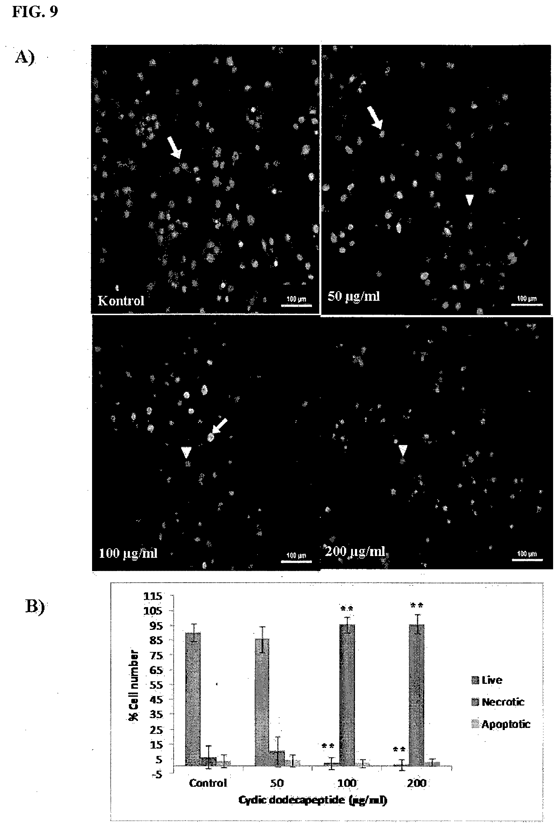

FIG. 9A is an image showing necrotic cells. Cyclic dodecapeptide increases the number of ethidium bromide dyed necrotic cell nucleus depending on dose in MCF-7 cells. White arrows indicate viable cell nucleus. Triangles indicate necrotic cell nucleus. Bar: 100 μm.

FIG. 9B is a graph showing cyclic dodecapeptide increased the necrotic cell number by significantly decreasing the viable cell number in MCF-7 cell relative to control group ($**p < 0,001$).

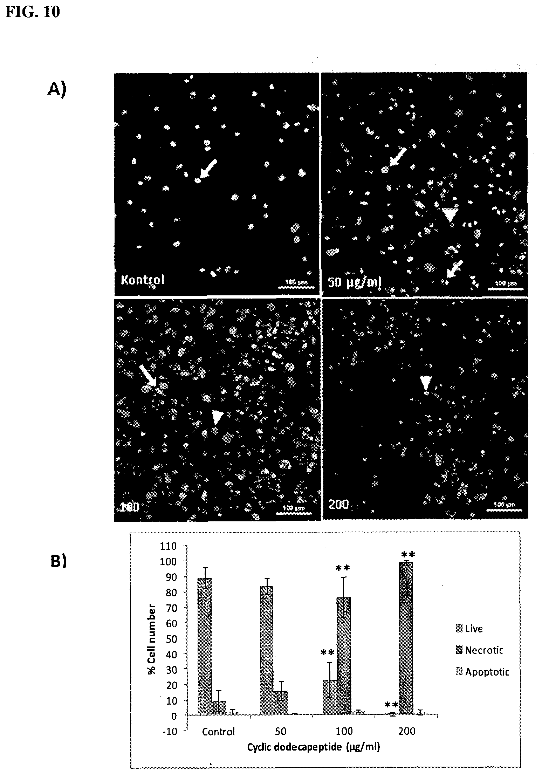

FIG. 10A is an image showing cyclic dodecapeptide increases the number of ethidium bromide dyed necrotic cell nucleus depending on dose in MDA-MB 231 cells. White arrows indicate viable cell nucleus. Triangles indicate necrotic cell nucleus. Shaded arrow indicates apoptotic cell nucleus. Bar: 100 μm. FIG. 10B is a graph showing cyclic dodecapeptide increased the necrotic cell number by significantly decreasing the viable cell number in MDA-MB 231 cell when compared to control group ($**p < 0,001$).

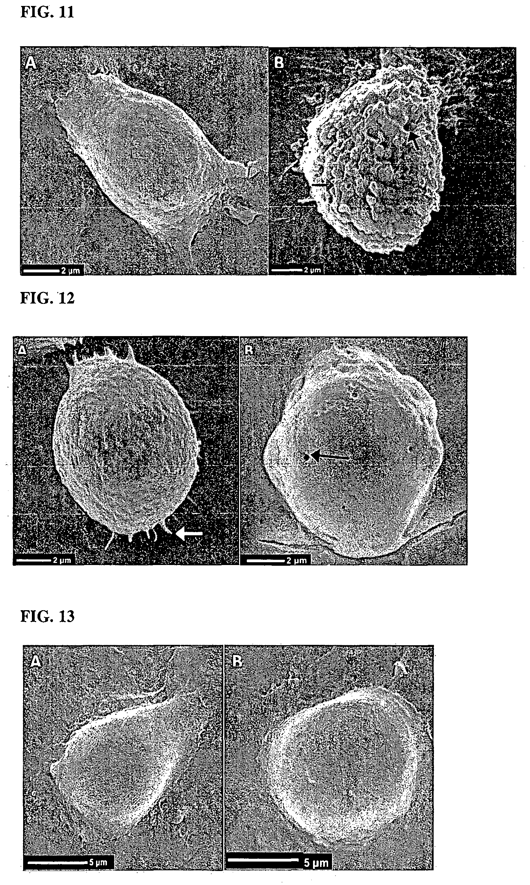

FIG. 11 is an image showing the effects of antimicrobial peptide on MCF-7 cell membrane. A) Control group; B) the group to which 100 μg/ml cyclic dodecapeptide is applied. Black arrow indicates pores. Shaded arrow indicates blebs.

FIG. 12 is an image showing the effects of antimicrobial peptide on

MDA-MB 231 cell membrane. A) Control group: B) the group to which 100 μg/ml cyclic dodecapeptide is applied. White arrow indicates microvillus. Black arrow indicates pores.

FIG. 13 is an image showing the effects of antimicrobial peptide on 3T3 fibroblast cell membrane. A) Control group; B) the group to which 100 μg/ml cyclic dodecapeptide is applied.

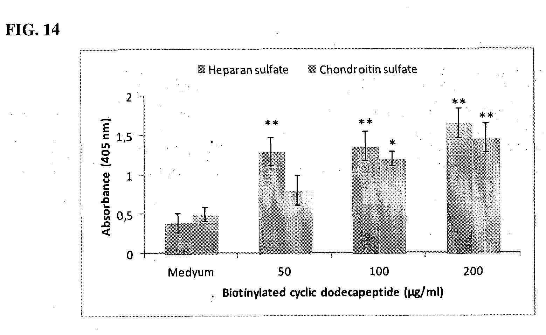

FIG. 14 is a graph showing the binding of biotin conjugated cyclic dodecapeptide to heparan sulfate and chondroitin sulfate. Significant increase was observed- when compared to medium ($*p < 0.05** p < 0.001$). Values of heparan sulfate: Medium: $0.394 \pm 0.12$, 50 μg/ml: $1,295 \pm 0.18$, 100 μg/ml: $1.364 \pm 0.18$, 200 μg/ml: $1.661 \pm 0.19$. Values of chondroitin sulfate: Medium: $0.494 \pm 0.09$, 50 μg/ml: $0.806 \pm 0.19$, 100 μg/ml: $1.207 \pm 0.09$. 200 μg/ml: $1.465 \pm 0.19$.

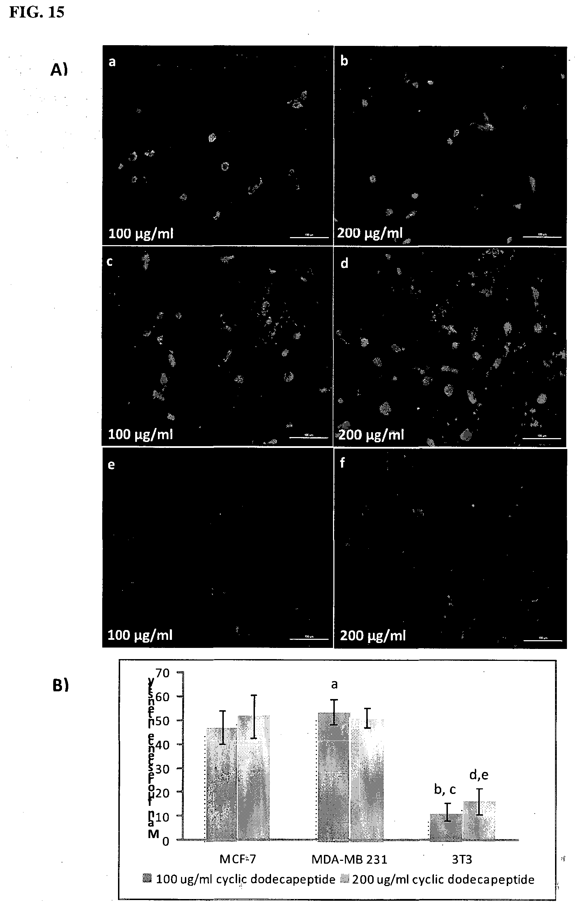

FIG. 15A is an image showing the comparison of affinities of cyclic dodecapeptide on MCF-7, MDA-MB 231 and 3T3 fibroblast cells. The binding of biotin-conjugated cyclic dodecapeptide to MCF-7, MDA-MB 231 and 3T3 fibroblast cells on fluorescent microscope, a and b: MCF-7 cells; c and d: MDA-MB 231 cells; c and f: 3T3 fibroblast cells. Bar: IOOμπι. FIG. 15B is a graph showing average fluorescent density values for MCF-7 100 μg/ml: $47.11 \pm 2.4$, 200 pg ml: $51,87 \pm 3.6$; for MDA-MB 231 100 μg/ml: $53.8 \pm 1.1$, 200 μg/ml: $51.24 \pm 1.1$: for 3T3 100 μg/ml: $11.75 \pm 0.8$, 200 μg/ml: $16.33 \pm 1.1$ a: significant when compared to 100 μg/ml cyclic dodecapeptide applied MCF-7 cells ($p < 0.05$); b: significant when compared to 100 μg/ml cyclic dodecapeptide applied MCF-7 cells ($p < 0.0001$); c: significant when compared to 100 μg ml cyclic dodecapeptide applied MDA-MB 231 cells ($p < 0.0001$); d: significant when compared to 200 μg/ml cyclic dodecapeptide applied MCF-7 cells ($p < 0.0001$); e: significant when compared to 200 μg/ml cyclic dodecapeptide applied MDA-MB 231 cells ($p < 0.0001$).

DETAILED DESCRIPTION OF THE INVENTION

The present invention relates to the medical use in the treatment of breast cancer of a cyclic dodecapeptide having the sequence ID No: 1 and comprising a disulfide between the two cysteine residues.

The following examples, methods or compositions arc not according to the invention and are present for illustration purposes only.

The invention features methods and pharmaceutical compositions for inducing cell death of cancerous cells in breast cancer using an antimicrobial peptide having a sequence of SEQ ID NO: 1, or a pharmaceutically acceptable salt thereof, in vitro or in vivo. The peptides are in cyclic form and have an overall positive charge. The peptides can selectively bind to the negatively charged molecules (e.g., negatively charged O-glycosylated mucin) on the cell surface of cancerous cells to induce cell death. The methods of the invention include administering therapeutically effective amounts of such peptides to breast cancer patients.

Antimicrobial peptides

Antimicrobial peptide used in the methods and pharmaceutical compositions of the invention contains 12 amino acids. Preferably, the peptide has an overall positive charge, which may be from 1 to 10. Preferably, the peptide has a high content of hydrophobic and basic amino acids. Hydrophobic amino acids include, but are not limited to, isoleucine, alanine, phenylalanine, valine, proline, tryptophan, and methionine. Basic amino acids include, but are not limited to, histidine, arginine, and lysine. In some embodiments, the peptide may be in a linear or cyclic form. Preferably, the peptide is in a cyclic form. The peptide may be in a cyclic form through disulfide bond formation between cysteine residues within the peptide. In other embodiments, a cysteine residue of a peptide may form a disulfide bond with a cysteine residue of the same or a different peptide of the invention, forming a homo-dimer or hetero-dimer of peptides. The two peptides in a homo-dimer or hetero-dimer may be in a parallel or anti-parallel form.

In some embodiments, the peptide used in the methods and pharmaceutical compositions of the invention has SEQ ID NO: 1 (RLCRIVVIRVCR). In a preferred embodiment of the invention, the peptide used in the methods and pharmaceutical compositions of the invention is a cyclic dodccapeptide that contains 12 amino acids (e.g., SEQ ID NO: 1 (RLCRIVVIRVCR)) and one disulfide bond formed between C3 and Cl 1 (underlined residues in the sequence of SEQ ID NO: 1).

Preferably, the peptide has a similar net charge, secondary structure, and hydrophobicity to the peptide with the sequence of SEQ ID NO: 1 (RLCRIVVIRVCR).

In a preferred embodiment of the invention, the peptides used in the methods and pharmaceutical compositions of the invention are produced using an automatic peptide synthesizer and purified with HPLC to at least 95% purity.

The peptide may selectively bind to the cell surface of breast cancer cells to induce cell death. Various mechanisms of cell death induction are described in detail further herein. The peptides used in the methods and pharmaceutical compositions of the invention may be used in vivo (e.g., in a subject (e.g., a human)) for breast cancer treatment.

Additionally, the peptides used in the methods and pharmaceutical compositions of the invention can be components of fusion proteins (e.g., fused to a tag for purification or to a protein that promotes stability in the blood (e.g., an Fc domain)). The peptides can include, e.g., one, two, three, four, or more additional amino acids at the N-terminal or C-terminal end of peptide of a sequence as described herein (e.g., SEQ ID NO: 1). In other embodiments, in the cases of cyclical peptides, additional amino acids can be located between any two amino acids (e.g., any two amino acids of SEQ ID NO: 1). Furthermore, the peptides can be conjugated to non-peptide molecules, including toxic compounds, chemotherapeutics (e.g., as described herein), or labels (e.g., fluorescent labels).

Mechanism of action of antimicrobial peptides in inducing cell death

Antimicrobial peptides have cytotoxic effects on cancer cells. This effect occurs via peptide-membrane interaction similar to peptide-bacterial membrane interaction. Most of the information about the cancer cell killing mechanism of the cationic antimicrobial peptides is acquired using artificial membranes or bacteria as model systems. However, various cellular models have different membrane compositions and transmembrane potentials. The characteristics of the target membrane, amino acid sequence, and secondary structure of the cationic antimicrobial peptides all determine whether certain cancer cells would be sensitive to antimicrobial peptidc-mediated cytotoxicity.

The first step to induce cell death is binding of the peptides to the target cell membrane. It has been stated that the electrostatic interaction between the positive charge present in the cationic antimicrobial peptides and the negatively charged surface molecules on the anionic target cell membrane is essential for this binding. The negatively charged cell surface of cancer cells originates from the negatively charged O-glycosylated mucins present on the outer leaflet of the cancer cell membrane. For example, sialylated gangliosides, negatively charged proteoglycans, such as heparan sulfate and chondroitin sulfate, and negatively charged phosphatidylserine are present on the cell surface of cancer cells three to nine times more than on the cell surface of normal, non-cancerous cells. On the outer leaflet of the cell membrane of normal cells, the presence of phosphatidylcholine and other zwitterionic phospholipids explain the decrease in binding affinity of cationic antimicrobial peptides to normal cells relative to cancerous cells. In some embodiments, it is suggested that several characteristics that are not currently supported by model membranes may contribute to the selective affinity of cationic antimicrobial peptides to cancer cells. For example, cancer cells have more negative transmembrane potential, larger surface area due to microvillus, and increased membrane viscosity. However, there is currently no study performed on model membranes to determine whether these characteristics contribute to the increased binding affinity of cationic microbial peptides to cancer cells. Several of these factors may contribute to peptide-mediated cytotoxicity. Furthermore, it is thought that the importance of each factor depends on the amino acid sequence and the secondary structure of the cationic antimicrobial peptide during interaction with the cell surface of cancer cells.

Most of the cationic antimicrobial peptides damage the cancer cell membranes in an irreparable way and directly kill the cancer cells. Some antimicrobial peptides can kill cancer cells indirectly without intense membrane damage. These peptides may induce apoptosis, inhibit macromolecule synthesis, and change receptor mediated signal transduction pathways.

Drug resistance is one of the most important problems causing failure in cancer treatments. Cationic antimicrobial peptides, such as peptides used in the methods and pharmaceutical compositions of the invention, e.g., peptides having sequence of SEQ ID NO: 1, may bind to breast cancer cells, including drug resistant cancer cells, and kill these cells without damaging vital organs. Furthermore, the slow growing cancer cells may be more sensitive to the cationic antimicrobial peptides than to chemotherapeutic drugs since the net charge of the cell surface determines the sensitivity of the cell to these peptides rather than the cell division rate.

The peptides used in the methods and pharmaceutical compositions of the invention, e.g.. peptides having sequence of SEQ ID NO: 1, may induce cell death of cancerous cells via one or more pathways depending on the cell type and type of cancer. For example, cell death by necrosis may be induced by pore formation on the cell surface of breast cancer cells caused by peptides used in the methods and pharmaceutical compositions of the invention.

Pharmaceutical compositions

In some embodiments, pharmaceutical compositions of the invention may contain one or more antimicrobial peptides (e.g., peptides having sequence of SEQ ID NO: 1) as the therapeutic peptides. In addition to a therapeutic amount of the peptide, the pharmaceutical compositions may contain a pharmaceutically acceptable carrier or excipient, which can be formulated by methods known to those skilled in the art. In a preferred embodiment, the pharmaceutical composition of the invention contains the peptide with the sequence of SEQ ID NO: 1 as the therapeutic peptide. Preferably, the peptide with the sequence of SEQ ID NO: 1 is in a cyclic form, e.g., a cyclic dodecapeptide. In some embodiments, the peptide may be used in a combination therapy, which includes co-administration (either together or separate) of one or more chemotherapeutic drugs for the treatment of cancers, e.g., breast cancer. In other embodiments, the peptide may be co-administered (either together or separate) with antioxidants to provide anti-inflammatory therapeutic benefits.

Acceptable carriers and excipients in the pharmaceutical compositions are nontoxic to recipients at the dosages and concentrations employed. Acceptable carriers and excipients may include buffers such as phosphate, citrate, HEPES, and TAE, antioxidants such as ascorbic acid and methionine, preservatives such as hexamethonium chloride, octadecyldimethylbcnzyl ammonium chloride, resorcinol, and benzalkonium chloride, proteins such as human serum albumin, gelatin, dextran, and immunoglobulins, polymers such as polyvinylpyrrolidone, polyethylene glycol, and polyvinyl,, amino acids such as glycine, glutamine, histidine, and lysine, and carbohydrates such as glucose, mannose, sucrose, lactose, dextrose, methyl cellulose, mannitol, and sorbitol. Other acceptable carriers and excipients may include physiologic saline, ethanol, isopropyl myristate, vegetable oils, polyethylene glycol, pyrrolidone, mineral oil, methylhydroxy benzoate, magnesium stearate, calcium silicate, gelatin, and propylhydroxy benzoate. Pharmaceutical compositions of the invention can be administered parenterally in the form of an injectable formulation. Pharmaceutical compositions for injection can be formulated using a sterile solution or any pharmaceutically acceptable liquid as a vehicle. Pharmaceutically acceptable vehicles include, but are not limited to, sterile water, physiological saline, and cell culture media (e.g., Du!becco's Modified Eagle Medium (DMEM), a-Modified Eagles Medium (a-MEM), F- 12 medium).

The pharmaceutical compositions of the invention may be prepared in microcapsules, such as hydroxylmethylcellulose or gelatin-microcapsule and poly-(methylmefhacrylate) microcapsules. The pharmaceutical compositions of the invention may also be prepared in other drug delivery systems such as liposomes, e.g., immunoliposomes, albumin microspheres. microemulsions, nano-particles, and nanocapsules. Such techniques are described in Remington: The Science and Practice of Pharmacy 20th edition (2000). The pharmaceutical compositions to be used for in vivo administration must be sterile. This is readily accomplished by filtration through sterile filtration membranes.

The pharmaceutical compositions of the invention may also be prepared as sustained-release formulations. Suitable examples of sustained-release preparations include semipermeable matrices of solid hydrophobic polymers containing the antimicrobial peptide(e.g., peptide having sequence of SEQ ID NO: 1). Examples of sustained release matrices include polyesters, hydrogels, polyactides (U.S. Patent No. 3,773,919), copolymers of L-glutamic acid and $\gamma$ ethyl-L-glutamate, non-degradable ethylene-vinyl acetate, degradable lactic acid-glycolic acid copolymers such as LUPRON DEPOT™, and poly-D-(-)-3-hydroxybutyric acid. Some sustained-release formulations enable release of molecules over a few months, e.g., one to six months, while other formulations release pharmaceutical compositions of the invention for shorter time periods, e.g., days to weeks.

The pharmaceutical composition may be formed in a unit dose form as needed. The amount of the peptide having a sequence of SEQ ID NO: 1, included in the pharmaceutical preparations is such that a suitable dose within a designated range is provided (e.g., a dose within the range of 0.01-100 mg/kg of body weight).

Routes, Dosage, and Timing of Administration

Pharmaceutical compositions of the invention that contain the antimicrobial peptide, (e.g.. peptide having sequence of SEQ ID NO: 1) as the therapeutic peptides may be formulated for, e.g., parenteral administration, intratumoral administration, subcutaneous administration, intracutaneous administration, intravenous administration, intracerebroventricular administration, intramuscular administration, intra-arterial administration, intratracheal administration, or interperitoneal administration.

In a preferred embodiment, the first in vivo administration of the antimicrobial peptide (e.g., peptide having sequence of SEQ ID NO: 1) may be intratumoral injection.

The antimicrobial peptide in the methods and pharmaceutical compositions of the invention may be used in vitro or in vivo (e.g., in a subject (e.g., a human)) for breast cancer treatment. The pharmaceutical compositions of the invention may also be formulated for, or administered via, nasal, spray, oral, aerosol, rectal, or vaginal administration. The pharmaceutical composition may also be administered via an epidural injection or sub-dermally via an infusion pump. The pharmaceutical compositions may also be administered as a cream or ointment for topical applications. Methods of administering therapeutic peptides are known in the art. See, for example, U.S. Patent Application Publication Nos. US20130259834 and US20100285003One or more of these methods may be used to administer a pharmaceutical composition of the invention that contains the antimicrobial peptide (e.g., peptide having sequence of SEQ ID NO: 1). For injectable formulations, various effective pharmaceutical carriers are known in the art. See, e.g., Pharmaceutics and Pharmacy Practice, J. B. Lippincott Company, Philadelphia, Pa., Banker and Chalmers, eds., pages 238-250 (1982), and ASHP Handbook on Injectable Drugs, Toissel, 4th ed., pages 622-630 (1986).

The dosage of the pharmaceutical compositions of the invention depends on factors including the route of administration, the form of the drug, the disease to be treated, and physical characteristics, e.g., age, weight, general health, of the subject. Typically, the amount of an antimicrobial peptide, e.g. a peptide having sequence of SEQ ID NO: 1, contained within a single dose may be an amount that effectively treats the disease without inducing significant toxicity. Preferably, the amount of the antimicrobial peptide, e.g., a peptide having sequence of SEQ ID NO: 1, contained within a single dose may be an amount that effectively treats breast cancer. A pharmaceutical composition of the invention may include a dosage of a peptide, e.g.. a peptide having a sequence of SEQ ID NO: 1, ranging from 0.001 to 500 mg/kg/day and, in a more specific embodiment about 0.01 to about 100 mg kg/day and, in a more specific embodiment, about 0.1 to about 20 mg/kg/day. The dosage may be adapted by the clinician in accordance with conventional factors such as the extent of the disease and different parameters of the subject. The therapeutically effective amount of an antimicrobial peptide in a pharmaceutical composition of the invention may also, be measured directly. Typically, a pharmaceutical composition of the invention can be administered in an amount from about 0.001 up to about 500 mg/kg/day (e.g., 0.001, 0.005, 0.01, 0.1, 0.2, 0.3, 0.5, 0.7, 0.8, 1, 2, 3, 4, 5, 10, 15, 20, 30, 50, 100, 250, or 500 mg/kg/day).

In a preferred embodiment, the dosage of the pharmaceutical compositions of the invention to be administered to xenografts with grown tumor mass may be from 0.01-0.5 mg/50 $\mu$l. The dosage may be administered as a single injection or multiple injections.

Pharmaceutical compositions of the invention that contain a peptide, e.g., a peptide having sequence of SEQ ID NO: 1, may be administered to a subject in need thereof, e.g., patients who have breast cancer, for example, one or more times (e.g., 1-10 times or more) daily, weekly, monthly, biannually, annually, or as medically necessary. Dosages may be provided in either a single or multiple dosage regimens. For example, in some embodiments, the effective amount is a dose that ranges from about 0.1 to about 100 mg/kg/day, from 0.2 to about 20 mg/kg/day. and from about 0.4 to about 40 mg/kg/day of the antimicrobial peptide, e.g., peptide having sequence of SEQ ID NO: 1, every other day. The timing between administrations may decrease as the medical condition improves or increase as the health of the patient declines.

Methods of Treatment

The invention provides methods and pharmaceutical compositions containing the antimicrobial peptide (e.g., peptide having sequence of SEQ ID NO: 1) that may be used to treat patients who are suffering from diseases and disorders, such as breast cancer.

The cancer amenable to treatment is breast cancer.

In some embodiments, the antimicrobial peptide (e.g., peptide having sequence of SEQ ID NO: 1) in the methods and pharmaceutical compositions of the invention may be used in a combination therapy, which includes co-administration (either together or separate) of one or more chemotherapeutic drugs for the treatment of cancers, e.g., breast cancer. In other embodiments, the antimicrobial peptides may be co-administered (either together or separate) with antioxidants to provide antiinflammatory therapeutic benefits.

In some embodiments, breast cancer cell lines may be used for in vitro study. Some examples of breast cancer cell lines are MCF-7 and MDA-MB 231 cell lines. The MCF-7 cell line is one of the most common and best characterized breast cancer lines. This cellular model has been used in studies to understand the roles of estrogen and antiestrogen in hormone dependant cancer cell proliferation. MCF-7 cells can create tumor in nude (without hair and thymus) mice when 17-$\beta$ oestradiol is applied. The MDA-MB 231 cell line is estrogen negative and carries p53 mutation. MDA-MB 231 cells are more invasive compared to MCF-7 cells. MDA-MB 231 cells can create tumor when they are injected into the fat pad of nude mice or create metastasis when they are injected into the tail vein.

EXAMPLES

Example 1 - Effects of cyclic dodecapeptide (SEQ ID NO: 1) on cell viability and toxicity MTT Assay

The MTT assay was used to measure cell viability. The cells were subjected to different concentrations of cyclic dodecapeptide (SEQ ID NO: 1) for 24 hours. Afterwards, 30 $\mu$l of the 3-(4,5-dimethyltiazol-2-yl)-2,5-diphcnyltctrazolium bromide (MTT) solution was added to each well on the plate. The MTT solution at 5 mg/ml was prepared in PBS at pH 7.4, and sterilized by filtering with membrane filter. Cells were incubated in MTT-containing media for 4 hours in an incubator with 5% carbon dioxide at 37 °C in the dark, and then the media was removed. The formazan crystals formed by reduction of MTT was dissolved by adding 100 µl DMSO into each well, and the plates were shaken in an orbital shaker for 5 minutes at 150 rpm. The optic density was measured at 570 nm test wavelength and 630 nm reference wavelengths.

Eight concentrations of cyclic dodecapeptide (SEQ ID NO: 1) were tested in this assay. Four wells of cells were used for each concentration of cyclic dodecapeptide. The, asay was performed in triplicates. Average absorbance values obtained from control group, which contain normal, non-cancerous cells, were treated as 100% cell viability. Average absorbance values obtained from the experiment group were evaluated relative to the absorbance values of the control group.

Treatment with cyclic dodecapeptide (SEQ ID NO: 1) decreased cell viability in both MCF-7 (FIG. 1) and MDA-MB 231 (FIG. 2) cell lines depending on the dose. The decrease in cell viability of MCF-7 cells was observed at 100 and 200 µg ml of cyclic dodecapeptide ($p < 0.001$) (FIG 1). The decrease in cell viability of MDA-MB 231 cells was observed starting at 50 µg/ml of cyclic dodecapeptide, and more significantly at 100 and 200 µg/ml of cyclic dodecapeptide ($p < 0.05$) (FIG. 2) These results indicate that that MDA-MB 231 cells appear to be more sensitive to cyclic dodecapeptide than MCF-7 cells.

The cell viability of 3T3 cells did not change relative to control groups at 2.5, 5, 7.5 and 10 µg/ml of cyclic dodecapeptide treatment. Cell viability of 3T3 cells increased at 25, 50, 100 and 200 µl /ml of cyclic dodecapeptide treatment (FIG.3).

Cytotoxicity Assay

The lactate dehydrogenase (LDH) release assay was used to measure cytotoxicity. Lactate dehydrogenase (LDH) is a stable cytoplasmic enzyme present in most cells. During damage to or rupture of cell cytoplasmic membranes, LDH is released from cells and into the surrounding cell culture supernatant. Quantitation of LDH in cell culture supernatant was used to measure cellular toxicity levels.

Both MCF-7 and control (normal) cells were detached from 75 cm$^2$ flasks with trypsin-EDTA and centrifuged. Cells were resuspended in cell culture media and diluted to a concentration of 50 cells/ µl 100 µl of cell suspension was transferred to each well of a 96-well plate. Three wells of cells were used for each concentration of cyclic dodecapeptide. The asay was performed in triplicates. The media of the control group (no cyclic dodecapeptide treatment) was replenished after 24 hours of incubation. The media of the experiment groups was replaced with media containing 50, 100, or 200 µg/ml of cyclic dodecapeptide. After the cells were incubated with cyclic dodecapeptide for 24 hours, the activity of released lactate dehydrogenase was detected following manufacture prototol. 5 µl of lysis solution (2% Triton-X-100) was added to each well. The plate was incubated at 37 °C for 15 minutes. 100 µt of freshly prepared reaction mixture containing catalyst (Diaphoresis NAD$^+$mixture) and dye solution (iodotetrazolium chloride (INT) and sodium lactate) was added to each well. After incubation in dark at room temperature for 30 minutes, the reaction was stopped by adding 50 µl of stopping solution (1N HCl) to each well. Plates were shaken in an orbital shaker for 10 seconds. Spectrophotometric absorbance was measured at 490 nm test wavelength and 690 nm reference wavelength. The results were determined as % cytotoxicity. The average absorbance value of the negative control group (no cyclic dodecapeptide added) was subtracted from each of the experiment groups. A positive control group for maximum LDH release was used as 100% cytotoxicity. The control and experiment groups were compared relative to the positive control group, and their cytotoxicities were measured by using the formula below:

$$\% \, Cytotoxicity = \frac{[Experiment - Negative \, Control]}{[Positive \, Control - Negative \, Control]} * 100$$

MCF-7 cells released LDH at 100 and 200 µg/ml of cyclic dodecapeptide (FIG. 4). Similarly, MDA-MB 231 cells released LDH at 50, 100, and 200 µg/ml of cyclic dodecapeptide (FIG. 5).

Example 2 - Effects of cyclic dodecapeptide (SEQ ID NO: 1) on caspase activity

Caspases are a family of cysteine proteases that play essential roles in apoptosis. Measurement of caspase-3 activity was used to determine whether cyclic dodecapeptide induced cell death is dependent on caspases.

MCF-7 and MDA-MB 231 cells were each plated in the wells of a 6-well plate. Each well contained approximated one million cells. After 24 hours, media in each well was replaced with serum-free DMEM-F12 media containing cyclic dodecapeptide at 50, 100, or 200 pgtml. Media of the control group (no cyclic dodecapeptide treatment) was replaced with fresh media that did not contain the cyclic dodecapeptide. After 24 hours of incubation with cyclic dodecapeptide. cells were detached with trypsin-EDTA, transferred to the falcon tubes, counted on thoma slide, and centrifuged for 3 minutes at 1500g. Cell culture supernatant was removed and the cell pellet was lysed in 50 $\mu$l of cold cell lysis tampon for 10 minutes on ice. After one minute centrifuge at 10.000 g and 4°C, cell lysate was collected and stored at -20°C. Caspases-3 enzyme activity was measured with

ApoTarget Caspase-3 Protease Assay kit (Invitrogen).

Before measuring caspase activity, protein concentration of the cell lysate was determined using Bradford Assay and following manufacture's instructions. After incubating the cell lysate in Bradford solution for 5 minutes, absorbance was measured on BioTek Microplate reader at 595 nm wavelength. The cell lysate was diluted such that there would be 200 $\mu$g of protein in each 50 $\mu$l of cell lysis tampon. 50 $\mu$l of 2X reaction tampon containing 10 mM dithiotreitol was added to 50 $\mu$l of cell lysate. Subsequently, 5 $\mu$l of 4 mM DEVD-pNA was added to this mixture and the samples were incubated for 2 hours at 37 °C in the dark.

DEVD-*p*NA was not added to negative control samples. The absorbance of the samples was measured on a BioTek microplate reader at 405 nm.

The cyclic dodecapeptide did not cause a significant change statistically in DEVD-*p*Na decomposition in the experiment groups relative to control groups (no cyclic dodecapeptide added) (p>0,05) (FIGS. 6 and 7). The decrease in cell viability that we previously observed with cyclic dodecapeptide does not take place via caspases-3 enzyme.

Example 3 - Analysis of DNA Fragmentation

Agarose gel electrophoresis was carried out to analyze DNA fragmentations that may occur in cyclic dodecapeptide-induced cell death. MCF-7 and MDA-MB 231, cells were each plated into the wells of a 6-well plate. Each well contained about one million cells. After 24 hours, media in each well was replaced with fresh media containing cyclic dodecapeptide at 50, 100. or 200 $\mu$g ml. Media of the control group (no cyclic dodecapeptide treatment) was replaced with fresh media that did not contain the cyclic dodecapeptide. After 24 hours of cyclic dodecapeptide treatment, cells were risned with calcium/magnesium-free PBS and detached using trypsin-EDTA. Cells were collected and centrifuged for 3 minutes at 4100 rpm. The cell pellet was resuspended in 200 $\mu$l PBS.

DNAeasy Blood & Tissue kit (Qiagen) was used to isolate DNA from the cells. 20 $\mu$l Proteinase K. and 4 $\mu$l RNase A were added into the cell suspension and the mixture was vortexed to homogeneity. After incubation for 2 minutes at room temperature, 200 $\mu$l tampon AL was added into the samples, which were vortexed again to homogeneity. Samples were further incubated in a shaker for 10 minutes at 56°C. Subsequently, 200 $\mu$l 96% ethanol was added to all samples, which were vortexed to homogeneity. The samples were then transferred to DNeasy Mini spin columns, which were centrifuged at room temperature for 1 minute at 8000 g. 500 $\mu$l tampon AW1 was added to the DNeasy mini spin columns, which were centrifuged at room temperature for 1 minute at 8000 g. This step was repeated for a second time and the columns were centrifuged at room temperature for 3 minute at 20,000 g. Each mini spin column was transferred into a clean 1.5 ml eppendorf tube. 50 $\mu$l tampon AE was added to each column, which was incubated for one minute at room temperature and centrifuged for 1 minute at room temperature at 8000 g to elute bound DNA. The DNA concentration in the elution tampon was measured by using a Nanodrop 2000c (Thermo Scientific) device. Eluted DNA with ratios of DNA to RNA greater than 1.75 were accepted as pure.

Isolated DNA was visualized on a 1% agarose gels. Cyclic dodecapeptide did not cause DNA fragmentation in MCF-7 and MDA-MB 231 cells (FIG. 8).

Example 4 - Detection of cell death by cell morphology

MCF-7 and MDA-MB 231 cells were each plated into the wells of a 24-well plate. Each well contained about 10,000 cells. After 24 hours, media in each well was replaced with fresh media containing cyclic dodecapeptide at 50, 100, or 200 Mg/ml. Media of the control group (no cyclic dodecapeptide treatment) was replaced with fresh media that did not contain the cyclic dodecapeptide. After 24 hours of cyclic dodecapeptide treatment, media was replaced with PBS solution containing acridine orange (Sigma) (100 $\mu$g/ml) and ethidium bromide (Sigma) (100 $\mu$g/ml).

Cells were visualized with a fluorescent microscope (Nikon DS5MCU2) using FITC/CY3 filter.

Cells were grouped as viable, apoptotic or necrotic according to the morphology of their nuclei. Cells with intact green nuclei were counted as viable. Cells with green or orange fragmented nuclei were counted as apoptotic. Cells with intact orange nuclei were counted as necrotic (FIGS. 9 and 10). Cyclic dodecapeptide increased the necrotic cell death in a dose-dependent manner in both MCF-7 and MDA-MB 231 cells.

Table 1: Average percentages of viable, necrotic, and apoptotic MCF-7 cells

| Concentrations of cyclic dodecapeptide | % Viable cell | % Necrotic cell | % Apoptotic cell |
|---|---|---|---|
| Control | 90.03±6.1 | 6.34±7.5 | 3.63±4.2 |

(continued)

| Concentrations of cyclic dodecapeptide | % Viable cell | % Necrotic cell | % Apoptotic cell |
|---|---|---|---|
| 50 µg/ml | 85.88±9 | 10.14±10.2 | 3.98±4.2 |
| 100 µg/ml | 2.15±3.8 | 95.7±5.2 | 2.15±2.8 |
| 200 µg/ml | 1.13±3.7 | 96.09±6.3 | 2.78±2.5 |

Table 2: Average percentages of viable, necrotic, and apoptotic MDA-MB 231 cells

| Concentrations of cyclic dodecapeptide | % Viable cell | % Necrotic cell | % Apoptotic cell |
|---|---|---|---|
| Control | 89.12±6.8 | 9.10±6.7 | 2.08±1.5 |
| 50 µg/ml | 83.71±5.4 | 15.66±5.9 | 0.63±0.5 |
| 100 µg/ml | 22.6±11.4 | 76.29±13.3 | 2.11±0.8 |
| 200 µg/ml | 0.08±1.3 | 98.51±1.2 | 1.41±1.6 |

Example 5 - Detection of cellular membrane damage using scanning electron microscope

MCF-7, MDA-MB 231, and 3T3 fibroblast cells were each grown and cultured on 12-mm diameter cover slips, which were placed into wells of a 24-well plate. Each coyer slip contained about 50,000 cells. After 24 hours, media in each well was replaced with fresh media containing cyclic dodecapeptide at 100 µg ml. After 24 hour cyclic dodecapeptide incubation, cells were washed twice with 0.1 M sodium cacodylate tampon (pH 7.2) and fixed with 2.5% glutaraldehyde in 0.1 M sodium cacodylate tampon for 2 hours. After first fixation, cells were washed with 0.1 M sodium cacodylate tampon and fixed for a second time with 1% osmium tetroxide in 0.2 M sodium cacodylate tampon for 30 minutes. Cells were washed with 0.2 M sodium cacodylate tampon and dehydrated in increasing percentage of ethanol. Cells were incubated in 30% alcohol (5 minutes, 3x), 50% alcohol (5 minutes, 3x), 70% alcohol (10 minutes, 2x), 85% alcohol (15 minutes, 2x), 95% alcohol (15 minutes, 2x), and 100% alcohol (10 minutes, 3x). Subsequently, the cover slips were air dried and coated with gold using BioRad SC 502 coating device. Cells were examined with Jeol Neoscope JCM-500 scanning electron microscope.

MCF-7 cells in the control group (no cyclic dodecapeptide treatment) appeared to have smooth cell surface (FIG. 11 A). After 24 hour incubation with 100 µg/ml cyclic dodecapeptide, pore formation (FIG. 1 IB, black arrow) on the cell membrane and bleb formation (FIG. 11B, shaded arrow) on the cell surface was observed.

MDA-MB 231 cells in the control group (no cyclic dodecapeptide treatment) appeared to have an uneven cell surface with a small number of microvillus (FIG. 11 A). After 24 hour incubation with 100 µg/ml cyclic dodecapeptide, MDA-MB 231 cells appeared to be swollen with the cell surface appearing more smooth. Pores (FIG. 12B, arrow) of various sizes were also observed on the cell surface. 3T3 fibroblast cells in the control group (no cyclic dodecapeptide treatment) appeared to have a smooth cell surface (FIG. 13 A). Cyclic dodecapeptide treatment did not cause any morphological change on the cell surface (FIG. 13B).

Example 6 - Electrostatic interactions of cyclic dodecapeptide with negative charged macromolecules and different cells

Glycoconjugates, such as heparan sulfate and chondroitin sulfate, contribute greatly to the overall negative charge of cancer cell membranes. Solid phase heparan sulfate and chondroitin sulfate binding assay was used to determine the electrostatic interaction between the cyclic dodecapeptide and glycoconjugates, such as heparan sulfate and chondroitin sulfate

The binding capacity of cyclic dodecapeptide to glycoconjugates, such as heparan sulfate and chondroitin sulfate, was measured using a modified method of enzyme-linked immunosorbent assay (ELISA) (Silvesiri et al., J. Immunol. 53: 282-289, 2001). 96-well plates were coated with 100 µl heparan sulfate and chondroitin sulfate solutions (10 µg/ml in PBS (pH 7.4)) at room temperature overnight. The wells were washed twice with PBS containing 0.05% Tween 20 (PBST). 100 µl media containing conjugate of biotin and cyclic dodecapeptide at 50, 100, or 200 µg/ml was added to each well and incubated for 2 hours at 37 °C. The wells were washed with PBST after incubation.

Avidin conjugated horseradish peroxidase (HRP) was diluted with PBST containing 0.5% bovine serum albumin (BSA) and added to each well. After incubation at room temperature for 2 hours, the wells were washed with PBST, 100 µl of 3,3',5,5'-Tetramethylbenzidine (TMB) substrate was added to each well. The reaction was stopped after 10 minutes

by adding 50 μl of 1 N hydrochloric acid (HCl) to each well. Absorbance was measured at 450 nm with Biotek microplate reader. FIG. 14 shows that conjugate of biotin and cyclic dodecapeptide interacts with heparan sulfate and chondroitin sulfate.

Example 7 - Measuring the interaction of cyclic dodecapeptide with MCF-7, MDA-MB 231, and 3T3 fibroblast cells:

In order to determine whether the selective toxicity of cyclic dodecapeptide towards breast cancer cells, such as MCF-7 and MDA-MB 231 cells, is related to the interaction between cyclic dodecapeptide and anionic molecules on the cell surface, the interaction of cyclic dodecapeptidc with different cells was measured.

MCF-7, MDA-MB 231, and 3T3 fibroblast cells were each plated on cover slips that were placed into the wells of a 24-well plate. Each well contained about 50.000 cells. After 24 hours, media in each well was replaced with fresh media containing biotin conjugated cyclic dodecapeptide at 100 or 200 μg/ml. Media of the control group (no cyclic dodecapeptide treatment) was replaced with fresh media that did not contain cyclic dodecapeptide. After 1 hour of cyclic dodecapeptide treatment, cells were fixed with 4% paraformaldehyde for 20 minutes and washed .three times with PBS (pH 7.4) for 10 minutes. Cells were then incubated with Texas Red conjugated streptavidin (Invitrogen) at 5 μg/ml in PBS for 45 minutes and washed three times with PBS. The cover slips were mounted onto slides with florescent mounting medium (Invitrogen) and images were taken with florescent microscope (Nikon DS5MCU2) using Cy3 filter. The average fluorescent density in each cell was detected using NIS-Elements (Nikon) software.

FIG. 15 shows that MCF-7, MDA-MB 231, and 3T3 fibroblast cells exhibited various degrees of affinity towards cyclic dodecapeptide ($p>0.05$). Cyclic dodecapeptide at 100 g/ml appeared to have stronger interaction with MDA-MB 231 cells than with MCF-7 cells ([a] $p<0.05$). Cyclic dodecapeptide at 200 μg/ml appeared to have similar interaction with MDA-MB 231 cells and MCF-7 cells (($p>0.05$). Cyclic dodecapeptide appeared to only interact weakly with 3T3 fibroblast cells.

## Claims

1.   A cyclic dodecapeptide having the sequence of Sequence ID No: 1 wherein said peptide comprises a disulfide bond formed between the two cysteine residues for use in a method of inducing cell death of cancerous cells of breast cancer, said method comprising administering a therapeutically effective amount of said peptide to the cancerous cells in vivo and said peptide selectively binds to negatively charged molecules on the cell surface of cancerous cells over the cell surface of non-cancerous cells and selectively induces cell death of cancerous cells while not inducing cell death in non-cancerous cells.

2.   The peptide for use of claim 1 wherein said therapeutically effective amount is from 0.001 to 500 mg/kg/day.

3.   The peptide for use of any one of claims 1-2, wherein said in vivo is in a human.

4.   The peptide for use of any one of claims 1-3, wherein said method further comprises administering a chemotherapeutic agent together with said peptide, wherein the chemotherapeutic agent is selected from the group consisting of cisplatin, carboplatin, oxaliplatin, bleomycin, mitomycin C, calicheamicins, maytansinoids, doxorubicin, idarubicin, daunorubicin, epirubicin, busulfan, carmustine, lomustine, semustine, thalidomide, lenalidomide, methotrexate, 6-mercaptopurine, fludarabine, 5-azacytidine, pentostatin, cytarabine, gemcitabine, 5-fluorouracil, hdroxyurea, etoposide, teniposide, topotecan, irinotecan, chlorambucil, cyclophosphamide, ifofosfamide, melphalan, bortezomib, vincristine, vinblastine, vinorelbine, paclitaxel and docetaxel.

## Patentansprüche

1.   Cyclisches Dodecapeptid mit Sequenz von Sequenz ID NO: 1, wobei das genannte Peptid eine zwischen den zwei Cysteinresten gebildete Disulfidbindung zur Verwendung in einem Verfahren für Induzierung vom Zelltod der Krebszellen des Brustkrebses umfasst, das genannte Verfahren die Verabreichung einer therapeutisch wirksamen Menge vom genannten Peptid an die Krebszellen in vivo umfasst, und das genannte Peptid anstelle der Zelloberfläche der nicht-krebsartigen Zellen an die negativ geladenen Moleküle auf der Zelloberfläche der Krebszellen selektiv bindet und den Zelltod der Krebszellen selektiv induziert, während es den Zelltod der nicht-krebsartigen Zellen nicht induziert.

2.   Peptid zur Verwendung nach Anspruch 1, wobei die genannte therapeutisch wirksame Menge von 0.001 bis 500 mg/kg/Tag ist.

3. Peptid zur Verwendung nach einem der Ansprüche 1-2, wobei in vivo in einem Menschen ist.

4. Peptid zur Verwendung nach einem der Ansprüche 1-3, wobei das genannte Verfahren ferner die Verabreichung eines Chemotherapeutikums zusammen mit dem genannten Peptid umfasst, wobei das Chemotherapeutikum aus einer Gruppe ausgewählt ist, die aus Cisplatin, Carboplatin, Oxaliplatin, Bleomycin, Mitomycin C, Calicheamicine, Maytansinoide, Doxorubicin, Idarubicin, Daunorubicin, Epirubicin, Busulfan, Carmustin, Lomustin, Semustin, Thalidomid, Lenalidomid, Methotrexat, 6-Mercaptopurine, Fludarabin, 5-Azacytidin, Pentostatin, Cytarabin, Gemcitabin, 5-Fluorouracil, Hydroxyharnstoff, Etoposid, Teniposid, Topotecan, Irinotecan, Chlorambucil, Cyclophosphamid, Ifofosfamid , Melphalan, Bortezomib, Vincristin, Vinblastin, Vinorelbin, Paclitaxel und Docetaxel besteht.

**Revendications**

1. Dodécapeptide cyclique ayant la séquence de Séquence ID N°: 1, dans lequel ledit peptide comprend une liaison disulfure formée entre les deux résidus cystéine pour utilisation dans une méthode d'induction de la mort cellulaire des cellules cancéreuses du cancer du sein, ladite méthode comprend l'administration d'une quantité thérapeutiquement efficace dudit peptide aux cellules cancéreuses in vivo et ledit peptide se lie sélectivement à des molécules chargées négativement sur la surface cellulaire des cellules cancéreuses au lieu de la surface cellulaire des cellules non cancéreuses et induit sélectivement la mort cellulaire des cellules cancéreuses alors qu'il n'induit pas la mort cellulaire dans les cellules non cancéreuses.

2. Peptide pour utilisation selon la revendication 1, dans lequel la quantité thérapeutiquement efficace est comprise entre 0.001 à 500 mg/kg/jour.

3. Peptide pour utilisation selon l'une quelconque des revendications 1-2, dans lequel ledit in vivo est chez l'homme.

4. Peptide pour utilisation selon l'une quelconque des revendications 1-3, dans lequel ladite méthode comprend en outre l'administration d'un agent chimiothérapeutique avec ledit peptide, dans lequel l'agent chimiothérapeutique est choisi dans le groupe constitué de cisplatine, carboplatine, oxaliplatine, bléomycine, mitomycine C, calichéamicines, maytansinoïdes, doxorubicine, idarubicine, daunorubicine, épirubicine, busulfan, carmustine, lomustine, semustine, thalidomide, lénalidomide, méthotrexate, 6-mercaptopurine, fludarabine, 5-azacytidine, pentostatine, cytarabine, gemcitabine, 5-fluorouracile, hdroxyurée, étoposide, téniposide, topotécan, irinotécan, chlorambucil, cyclophosphamide, ifofosfamide, melphalan, bortezomib, vincristine, vinblastine, vinorelbine, paclitaxel et docétaxel.

FIG. 1

FIG. 2

FIG. 3

FIG. 4

**FIG. 5**

**FIG. 6**

**FIG. 7**

**FIG. 8**

FIG. 9

A)

B)

FIG. 10

A)

B)

**FIG. 11**

**FIG. 12**

**FIG. 13**

**FIG. 14**

**FIG. 15**

**A)**

**B)**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9960016 A
- US 3773919 A
- US 20130259834 A
- US 20100285003 A

**Non-patent literature cited in the description**

- **GASPAR.** From antimicrobial to anticancer peptide. A review. *Fromtiers in Microbiology,* 2013, vol. 4 (294), 1-16
- **HOSKIN.** Studies on anticancer activities of antimicrobial peptides. *Biochimica et Biophysica Acta (BBA) - Biomembranes,* 2007, vol. 1778 (2), 357-375
- **MADER.** Cationic antimicrobial peptides as novel cytotoxic agents for cancer treatment. *Expert Opnion on Investigational Drugs.,* 2006, vol. 15 (8), 933-946
- **RADERMACHER.** Bactenecin, a leukocytic antimicrobial peptide, is cytotoxic to neuronal and glial cells. *Journal of Neuroscience Research,* 1993, vol. 36 (6), 657-662
- Remington: The Science and Practice of Pharmacy. 2000
- Pharmaceutics and Pharmacy Practice. J. B. Lippincott Company, 1982, 238-250
- ASHP Handbook on Injectable Drugs. Toissel, 1986, 622-630
- **SILVESIRI et al.** *J. Immunol.,* 2001, vol. 53, 282-289